(19) Europäisches Patentamt
European Patent Office
Office européen des brevets

(11) Veröffentlichungsnummer: **0 046 188**
**B1**

(12) **EUROPÄISCHE PATENTSCHRIFT**

(45) Veröffentlichungstag der Patentschrift: **03.04.85**

(51) Int. Cl.[4]: **C 07 D 498/08** // (C07D498/08, 261:00, 231:00)

(21) Anmeldenummer: **81105445.1**

(22) Anmeldetag: **13.07.81**

(54) **Verfahren zur Herstellung von Isoxazolino-azo-Verbindungen.**

(30) Priorität: **20.08.80 DE 3031403**

(43) Veröffentlichungstag der Anmeldung:
**24.02.82 Patentblatt 82/08**

(45) Bekanntmachung des Hinweises auf die Patenterteilung:
**03.04.85 Patentblatt 85/14**

(84) Benannte Vertragsstaaten:
**BE CH DE FR GB LI**

(56) Entgegenhaltungen:
**TETRAHEDRON LETTERS, No. 34, 1978, Oxford JÄGER "Syntheses via Isoxazolines" Seiten 3129-3136**
**J. Org. Chem. 41 (1976) S. 403-419**
**Liebigs Ann.443 (1925) S. 242-256**

(73) Patentinhaber: **BASF Aktiengesellschaft**
**Carl-Bosch-Strasse 38**
**D-6700 Ludwigshafen (DE)**

(72) Erfinder: **Rieber, Norbert, Dr.**
**Liebfrauenstrasse 1C**
**D-6800 Mannheim (DE)**
Erfinder: **Boehm, Heinrich, Dr.**
**Keplerweg 2**
**D-6701 Neuhofen (DE)**
Erfinder: **Platz, Rolf, Dr.**
**Hansastrasse 5**
**D-6800 Mannheim (DE)**
Erfinder: **Fuchs, Werner, Dr.**
**Muenchbuschweg 30B**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 046 188 B1

**Beschreibung**

Die Erfindung betrifft ein Verfahren zur Herstellung von Isoxazolino-azo-verbindungen durch Umsetzung von Nitriloxiden mit Diaza-bicycloheptenen und anschließender Oxidation der erhaltenen N,N-Dicarbalkoxy-isoxazolino-hydrazo-verbindungen in Gegenwart basischer Verbindungen unter Verwendung von Alkalihalogeniten oder Wasserstoffperoxid.

Es ist aus Tetrahedron Letters 1978, Seiten 3 129 bis 2 136, bekannt, daß man Nitriloxide mit offenkettigen Alkenen oder Cyclopentenen zu Isoxazolinen umsetzen kann. Entsprechende Cyclisierungen, auch zu Isoxazolinen, durch Addition von Nitriloxiden, Nitriliminen und Nitronen an Olefine zeigt J. Org. Chem. *41* (1976), Seiten 403—419.

Es ist aus Liebigs Annalen der Chemie, Bd, 443, Seiten 242—256 (1925) bekannt, daß man Cyclopentadien an Azoester addieren, das gebildete Additionsprodukt hydrieren kann und dann durch Verseifung des Hydrierungsprodukts mit methanolischer Kalilauge des Endo-methylen-piperidazin erhält. Behandelt man dieses Piperidazin mit einer wäßrigen Lösung von Cuprihalogeniden, wird es zu Endo-methylen-dehydropiperidazin umgewandelt, das mit dem gebildeten Cuprohalogenid in eine schwer lösliche Molekülverbindung übergeht.

Auch JACS, Bd. 91 (1969), S. 5668—5669, beschreibt unter Hinweis auf die vorgenannte Veröffentlichung in Liebigs Annalen die Verseifung und Decarboxylierung in methanolischer Kalilauge und eine Oxidation mit Cuprichlorid zu einer Cu-I-Molekülverbindung.

Es wurde nun gefunden, daß man Isoxazolino-azo-verbindungen der Formel (I)

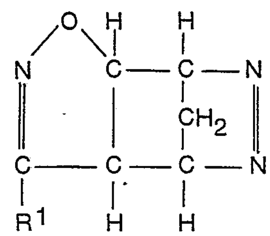

I

worin $R^1$ ein Wasserstoffatom, einen gegebenenfalls durch eine Hydroxygruppe substituierten Alkylrest mit 1 bis 18 Kohlenstoffatomen, einen Cycloalkylrest mit 3 bis 8 Kohlenstoffatomen, einen Aralkylrest oder Alkylarylrest mit jeweils 7 bis 12 Kohlenstoffatomen oder einen unsubstituierten oder einen Phenyl — oder Naphthylrest bezeichnet, der 1—3 fach durch Fluor, Brom, Chlor, Nitro, Trifluormethyl, Hydroxy, Dialkylamino, Alkoxy und/oder durch Fluor substituiertes Alkoxy mit jeweils 1 bis 4 Kohlenstoffatomen je Alkylgruppe substituiert sein kann, vorteilhaft erhält, wenn man in einem ersten Schritt Nitriloxide der Formel (II)

$$R^1CNO \qquad II,$$

worin $R^1$ die vorgenannte Bedeutung besitzt, mit N,N'-Dicarbalkoxy-2,3-diaza-bicyclo-[2,2,1]-hept-5-enen der Formel (III)

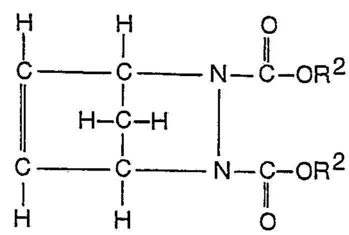

III

worin die einzelnen Reste $R^2$ gleich oder verschieden sein können und jeweils ein Wasserstoffatom oder einen gegebenenfalls durch eine Hydroxygruppe substituierten Alkylrest mit 1 bis 18 Kohlenstoffatomen bedeuten, umsetzt und dann in einem 2. Schritt die so erhaltenen N,N'-Dicarbalkoxy-isoxazolino-hydrazo-verbindungen der Formel (IV)

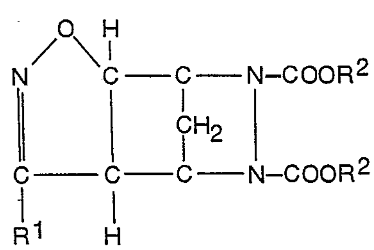

IV

worin $R^1$ und $R^2$ die vorgenannte Bedeutung besitzen, in Gegenwart eines Lösungsmittels, bestehend aus Wasser, Alkanolen und/oder Cycloalkanolen, und bei 40—100°C mit 4—25 Äquivalenten einer basischen Alkaliverbindung behandelt und gleichzeitig oder anschließend mit Alkalihypohalogeniten oder Wasserstoffperoxid oxidiert.

Die Umsetzung kann für den Fall der Verwendung von Methylnitriloxid, N,N-Dicarbmethoxy-2,3-diazabicyclo-2,2,1-hept-5-en, Wasserstoffperoxid und Natronlauge durch die folgenden Formeln beschrieben werden:

1) 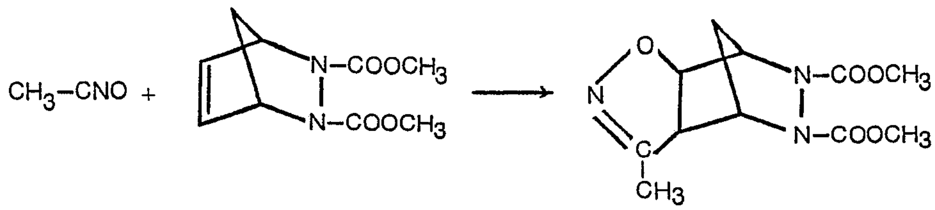

2) 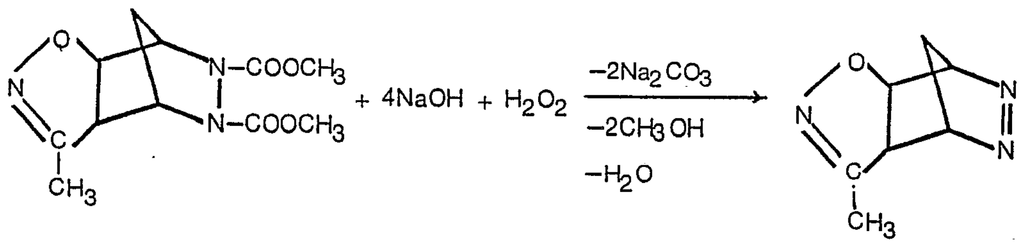


Das Verfahren nach der Erfindung liefert auf einfachem und wirtschaftlichem Wege die neuen Isoxazolino-azo-verbindungen in guter Ausbeute und Reinheit. Im Hinblick auf den Stand der Technik (Liebigs Annalen, JACS, loc. cit.) war es überraschend, daß die Herstellung des Endstoffs ohne Cuprihalogenid auf anderem Wege, nämlich mit Alkalihypohalogeniten oder Wasserstoffperoxid durchgeführt werden kann.

Die Ausgangsstoffe (II) sind leicht durch die in Houben-Weyl, Methoden oder Organischen Chemie, Band 10/3, Seiten 841 bis 853 beschreibenen Arbeitsweisen, z.B. durch Dehydrierung von Aldoximen oder aus Hydroxamsäurederivaten oder Nitrolsäuren zugänglich. Die Ausgangsstoffe (III) erhält man z.B. durch Umsetzung von Cyclopentadien mit Azodicarbonsäuredimethylester nach den in Ann. *443*, 242—256 (1925) beschriebenen Verfahren.

Bevorzugte Ausgangsstoffe (II) und (III) und dementsprechend bevorzugte Stoffe (IV) und Endstoffe (I) sind solche, in deren Formeln die einzelnen Reste $R^1$ und $R^2$ gleich oder verschieden sein können und jeweils ein Wasserstoffatom oder einen gegebenenfalls durch eine Hydroxygruppe substituierten Alkylrest mit 1 bis 12, insbesondere 1 bis 4 Kohlenstoffatomen bedeuten. Alle Ausgangsstoffe II werden zweckmäßig im ersten Verfahrensschritt in situ aus ihren Vorprodukten, z.B. aus den Oximen der Formel (V)

$$R^1\text{—CH} = \text{N—OH} \qquad \text{V}$$

durch Dehydrierung oder den Hydroxamsäurehalogeniden der Formel (VI)

$$\begin{array}{c} R^1\text{—C} = \text{N—OH} \\ | \\ \text{X} \end{array} \qquad \text{VI}$$

worin $R^1$ die vorgenannte Bedeutung besitzt und X ein Halogenatom, vorzugsweise ein Bromatom oder Chloratom, bezeichnet, durch Halogenwasserstoffabspaltung unter Verwendung basischer Stoffe hergestellt.

Es kommen z.B. als Ausgangsstoffe (II) in Betracht: Methyl-, Äthyl-, Propyl-, Isopropyl-, Butyl-, sek.-Butyl-, tert.-Butyl-, Isobutyl-, Pentyl-, Hexyl-, Heptyl-, Octyl-, Nonyl-, Decyl-, Undecyl-, Dodecyl-, Cyclohexyl-, Cyclopentyl-, Benzyl-, Phenyl-, 2-Chlorphenyl-, 3-Chlorphenyl-, 4-Chlorphenyl-, 2,4-Dichlorphenyl-, 2-Bromphenyl-, 3-Bromphenyl- oder 4-Bromphenyl-, o-Methylphenyl-, m-Methylphenyl-, p-Methylphenyl-, Naphthyl-nitriloxid, Cyclopropyl, o-Methoxyphenyl-, m-Methoxyphenyl-, p-Methoxyphenyl-nitriloxid.

Als Ausgangsstoffe (III) kommen in Frage: N,N'-Dicarbmethoxy-2,3-diaza-bicyclo-[2,2,1]-hept-5-en; homologe Diäthyl-, Dipropyl-, Diisopropyl-, Dibutyl-, Diisobutyl-, Di-sek.-butyl-, Di-tert.-butyl-ester vorgenannter Heptene.

Die Umsetzung wird vorteilhaft in der ersten Stufe bei einer Temperatur von −25 bis 100°C, vorzugsweise von 0 bis 40°C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen z.B. in Frage: Halogenkohlenwasserstoffe, insbesondere Brom- und Chlorkohlenwasserstoffe, z.B. Tetrachloräthylen, 1,1,2,2- oder 1,1,1,2-Tetrachloräthan, Amylchlorid, Cyclohexylchlorid, 1,2-Dichlorpropan, Methylenchlorid, Dichlorbutan, Isopropylbromid, Chlornaphthalin, Dichlornaphthalin. Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, Chlorbenzol, Fluorbenzol, Brombenzol, Jodbenzol, o-, p- und m-Dichlorbenzol, o-, p-, m-Dibrombenzol, o-, m- p-Chlortoluol, 1,2,4-Trichlorbenzol, 1,10-Dibromdekan, 1,4-Dibrombutan; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thioanisol, ß,ß'-Dichlordiäthyläther; und entsprechende Gemische. Zweckmäßig verwendet man das Lösungsmittel in einer Menge von 100 bis 10 000 Gewichtsprozent, vorzugsweise von 100 bis 2 000 Gewichtsprozent, bezogen auf Ausgangsstoff (II).

Die Reaktion des ersten Schrittes kann wie folgt durchgeführt werden: Ein Gemisch der Ausgangsstoffe (II) und (III) und zweckmäßig Lösungsmittel wird während 3 bis 22 Stunden bei der Reaktionstemperatur gehalten. Der Ausgangsstoff (II) wird in situ hergestellt, indem man z.B. das zugrundeliegende Oxim und ein Oxidationsmittel, z.B. Natriumhpochloritlösung, oder z.B. das zugrundeliegende Hydroxamsäurechlorid und ein tertiäres Amin, z.B. Triäthylamin, zugibt. Dann wird der Stoff (IV) aus dem Gemisch nach bekannten Methoden, z.B. durch Filtration oder Extraktion und fraktionierte Destillation, abgetrennt.

Der 2. Schritt der Umsetzung wird mit einer basischen Alkaliverbindung in einer Menge von 4 bis 25, vorzugsweise von 4 bis 8 Äquivalenten basischer Verbindung, bezogen auf ein Mol Ausgangsstoff (II), durchgeführt. Bevorzugte basische Verbindungen sind wäßrige oder alkoholische Natron- oder Kalilauge, sowie entsprechende Gemische. Es kommen z.B. als basische Verbindungen in Frage Kaliumhydroxid, Natriumhydroxid, Kaliumcarbonat, Natriumcarbonat, Lithiumhydroxid, Lithiumcarbonat, Natriumbicarbonat, Kaliumbicarbonat.

Die Oxidation erfolgt in der 2. Stufe mit Alkalihypohalogeniten oder Wasserstoffperoxid. Das Wasserstoffperoxid wird zweckmäßig in Form seiner 1- bis 90-, vorzugsweise 20- bis 55-gewichtsprozentigen, wäßrigen Lösung verwendet. Gegebenenfalls kommen auch Stoffe in Betracht, die unter den Umsetzungsbedingungen Wasserstoffperoxid bilden, z.B. anorganische Peroxoverbindungen wie Natriumperoxid, Kaliumperoxid, Magnesiumperoxid, Calciumperoxid, Zinkperoxid, Bariumperoxid, Bariumsuperoxid; Hydroperoxide wie $NaOOH \cdot 0{,}5\ H_2O_2$, $NH_4OOH$; entsprechende Hydrate wie $CaO_2 \cdot 8H_2O$, Peroxohydrate wie $BaO_2 \cdot H_2O_2$ und $BaO_2 \cdot 2\,H_2O_2$; Peroxocarbonate wie Natriumperoxocarbonat und Calciumperoxocarbonat; Peroxophosphate wie das Kaliumperoxodiphosphat. Ebenfalls können Wasserstoffperoxidanlagerungsverbindungen verwendet werden wie Natriumboratperoxohydrat. Gegebenenfalls können Hilfsstoffe wie Magnesiumsulfat oder Magnesiumchlorid zugesetzt werden.

Als Alkalihypohalogenite verwendet man vorteilhaft in der 2. Verfahrensstufe Hypochlorite und Hypobromite in wäßrigem Medium, in der Regel in Gestalt von entsprechenden wäßigen, alkalischen Lösungen. Die wäßrigen Alkalihypohalogengenitlösungen, vorzugsweise Hypochloritlösungen, enthalten im allgemeinen von 5 bis 25, vorzugsweise von 12 bis 14 Gewichtsprozent Hypohalogenit, vorzugsweise Hypochlorit, und können zweckmäßig zusätzlich von 0,2 bis 2,5 Mol, vorzugsweise 0,5 bis 2,1 Mol Alkalihydroxid je Mol Hypohalogenit, vorzugsweise Hypochlorit, enthalten. Im Ausgangsgemisch beider Ausgangsstoffe kommen im allgemeinen Mengen von 0,9 bis 2,5, vorzugsweise 0,95 bis 1,1 mol Hypohalogenit, zweckmäßig Hypochlorit, und zweckmäßig von insgesamt 4 bis 25 Mol, vorzugsweise 4 bis 8 Mol Alkalihydroxid (nicht eingerechnet das im Hypohalogenit enthaltene Alkali), bezogen auf ein Mol Ausgangsstoff (II), in Frage. Bevorzugte Alkalihypohalogenite sind das Natrium- oder das Kaliumsalz.

Die Umsetzung der 2. Stufe wird bei einer Temperatur von 40 bis 100°C, drucklos oder unter Druck, kontinuierlich oder diskontinuierlich in Gegenwart eines Lösungsmittels bestehend aus Wasser Alkanolen und oder Cycloalkanolen durchgeführt. Beispiele für Lösungsmittel sind Äthanol, Methanol, n-Butanol, Isobutanol, tert.-Butanol, Glykol, Glycerin, n-Propanol, Isopropanol, Amylalkohol, Cyclohexanol; und entsprechende Gemische. Zweckmäßig verwendet man das Lösungsmittel in einer Menge von 100 bis 10 000 Gewichtsprozent, vorzugsweise von 100 bis 1 000 Gewichtsprozent, bezogen auf Ausgangsstoff (II).

Die Reaktion des 2. Schrittes kann wie folgt durchgeführt werden: Ein Gemisch von Stoff (IV), basischer Alkaliverbindung, Oxidationsmittel und Lösungsmittel, wird während 2 bis 8 Stunden bei der Reaktionstemperatur des 2. Schrittes gehalten. In der Regel wird man zunächst Stoff (IV), die basische Alkaliverbindung und der Lösungsmittel einige Zeit, zweckmäßig 1 bis 5 Stunden, bei der Reaktionstemperatur des 2. Schrittes halten, dann das Oxidationsmittel, zweckmäßig zusammen mit einen weiteren Anteil an Lösungsmittel, zusetzen und die Reaktion, zweckmäßig während 1 bis 7 Stunden, durchführen. Aus dem Reaktionsgemisch wird der Endstoffe (I) in üblicher Weise, z.B. durch Filtration, Extraktion, Einengen des Filtrats und Filtration, abgetrennt.

Die nach dem Verfahren der Erfindung erhältlichen Isoxazolino-azo-verbindungen sind wertvolle Lichtschutzmittel, besonders im UV—B—Bereich und wertvolle Ausgangsstoffe für die Herstellung von Farbstoffen, Schädlingsbekämpfungsmitteln, Lichtschutzmitteln und Pharmazeutika. Sie absorbieren in dem für die Bräunung der Haut schädlichen UV—B und C—Bereich (215 — 315 nm) der natürlichen UV—Strahlung. Der Absorptionsbereich der Verbindungen liegt zwischen 215 und 315. Bezüglich der allgemeinen Eigenschaften und Verwendung von Lichtschutzmitteln wird auf Naturwissenschaftliche Rundschau, Band 25, Seiten 89 bis 99 (1972) verwiesen. Ebenfalls können aus den Endstoffen I auch andersartig substituierte Isoxazoline hergestellt werden. Die Endstoffe (I) können zur Herstellung von Prostaglandinen verwendet

werden, deren Eigenschaften in New Scientist, *69*, No. 981, Seiten 9 bis 12 (1976) beschrieben werden. Bezüglich der Verwendung wird auf vorgenannte Veröffentlichungen verwiesen.

Die im folgenden angegebenen Teile bedeuten Gewichtsteile.

Beispiel 1

a)

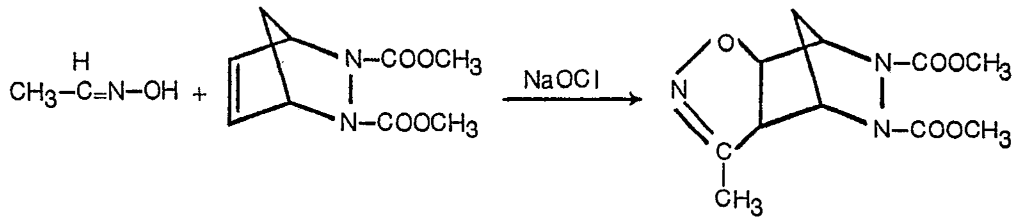


Zu 702 Teilen N,N'-Dicarbmethoxy-2,3-diaza-bicyclo-2,2,1-hept-5-en und 200 Teilen Acetaldoxim in 3562 Teilen Methylenchlorid gibt man innerhalb von 3 Stunden unter Rühren bei 10°C 2742 Teile 13-gewichtsprozentige, wäßrige Natriumhypochloritlösung zu und rührt noch eine Stunde bei 10°C nach. Anschließend wird die organische Phase abgetrennt und die wäßrige Phase dreimal mit 200 Teilen $CH_2Cl_2$ extrahiert. Die vereinigten organischen Phasen werden im Rotationsverdampfer eingeengt und der Rückstand mit 500 Teilen Petroläther digeriert. Man erhält 829 Teile (93% der Theorie) 8-Methyl-N,N'-dicarbmethoxy-2,3,7-triaza-6-oxa-tricyclo-[5.2.1.$0^{5.9}$]-deca-7-en vom Fp 124°C.

b)

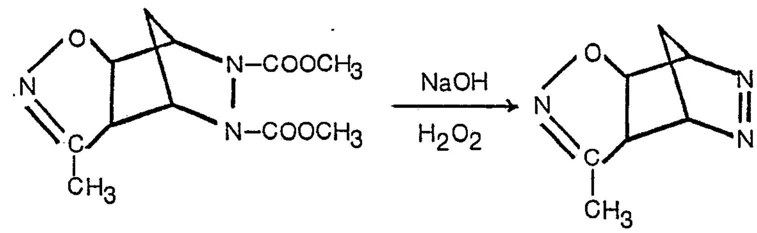


350 Teile 8-Methyl-N,N'-dicarbmethoxy-2,3,7-triaza-6-oxatricyclo-[5.2.1.$0^{5.9}$]-deca-7-en werden bei 70°C 4 Stunden mit 850 Teilben 25-gewichtsprozentiger Natronlauge gerührt. Danach gibt man bei 70°C 88 Teile $H_2O_2$ in 792 Teilen Wasser innerhalb von 2 Stunden unter Rühren zu und rührt noch eine Stunde bei 70°C nach. Anschließend wird auf 25°C gekühlt, der Feststoff abgesaugt und mit 100 Teilen Wasser gewaschen. Die Filtrate werden dreimal mit 300 Teilen $CH_2Cl_2$ extrahiert Die organische Phase wird im Rotationsverdampfer eingeengt. Der abgesaugte Feststoff und der Rückstand aus der organischen Phase werden vereinigt und getrocknet. Man erhält 175 Teile (89% der Theorie) 8-Methyl-2,3,7-triaza-6-oxo-tricyclo-[5.2.1.$0^{5.9}$]-deca-2,7-dien vom Fp 76°C.

Beispiel 2

a)

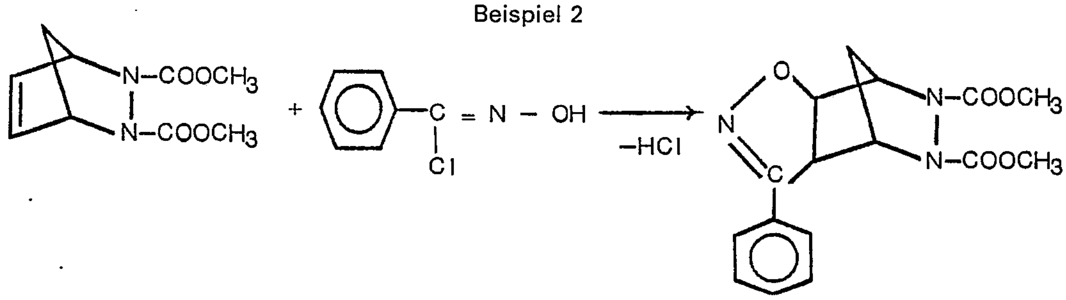


Zu 212 Teilen N,N'-Dicarbmethoxy-2,3-diaza-bicyclo-[2.2.1]-hept-5-en in 2 000 Teilen Äther und 110 Teilen Triäthylamin gibt man innerhalb einer Stunde bei 25°C unter Rühren 155,5 Teile Benzhydroxamsäurechlorid und rührt noch 20 Stunden bei 25°C nach. Der ausgefallene Endstoff wird abgesaugt, mit Wasser alkalifrei gewaschen und getrocknet. Man erhält 284,5 Teile (86% der Theorie) 8-Phenyl-N,N'-dicarbmethoxy-2,3,7-triaza-6-oxa-tricyclo-[5.2.1.$0^{5.9}$]-deca-7-en vom Fp 173°C.

b)

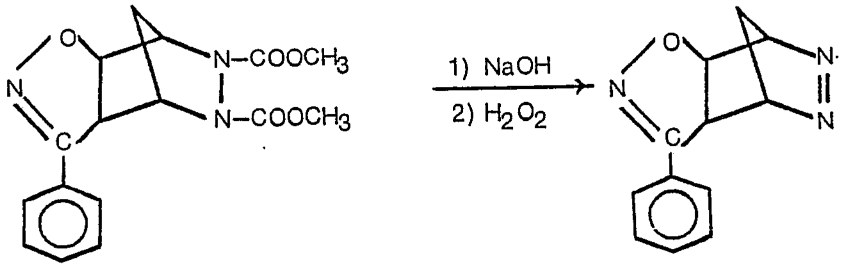

420 Teile 8-Phenyl-N,N'-dicarbmethoxy-2,3,7-triaza-6-oxatricyclo-[5.2.1.$0^{5,9}$]-deca-7-en werden bei 68°C 4 Stunden mit 505 Teilen 50-gewichtsprozentiger Natronlauge und 2 000 Teilen $CH_3OH$ gerührt. Danach gibt man bei 68°C 300 Teile 30-gewichtsprozentige, wäßrige $H_2O_2$-Lösung innerhalb von 2 Stunden unter Rühren zu und rührt noch eine Stunde bei 68°C nach. Anschließend versetzt man das auf 25°C abgekühlte Reaktionsgemisch mit 1 000 Teilen Wasser, saugt den Niederschlag ab und wäscht ihn mit Wasser alkalifrei. Die vereinigten Filtrate werden mit 3 × 300 Teilen $CH_2Cl_2$ extrahiert; das Methylenchlorid wird im Rotationsverdampfer abdestilliert. Der abgesaugte Feststoff und der Rückstand aus der $CH_2Cl$-Phase werden vereinigt und getrocknet. Man erhält 246 Teile (91% der Theorie) 8-Phenyl-2,3,7-triaza-6-oxa-tricyclo-[5.2.1.$0^{5,9}$]-deca-2,7-dien vom Fp 126°C.

Beispiel 3

a)

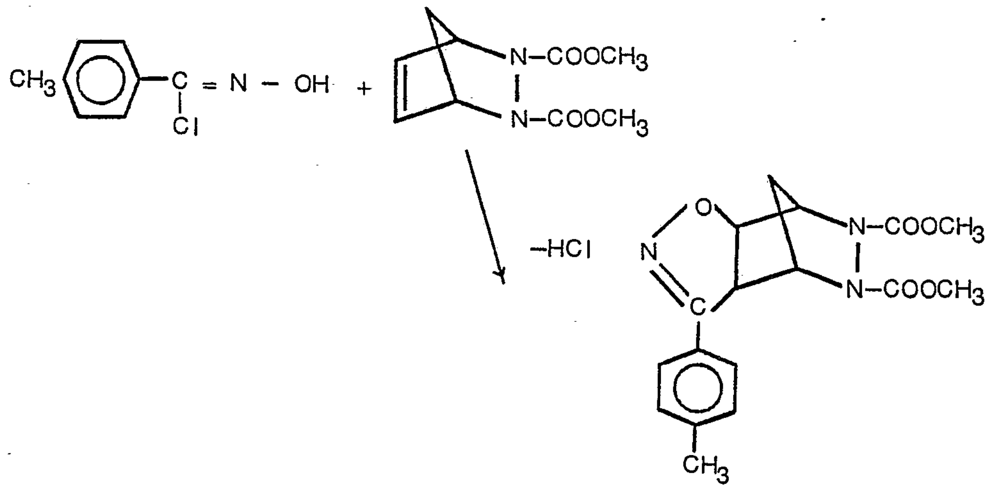


Zu 86 Teilen N,N'-Dicarbmethoxy-2,3-diaza-bicyclo-2,2,1-hept-5-en und 70 Teilen Triäthylamin in 800 Teilen Äther gibt man unter Rühren innerhalb von einer Stunde bei 25°C 69 Teile p-Methylbenzhydroxamsäurechlorid und rührt 22 Stunden bei 25°C nach. Anschließend wird der Niederschlag abgesaugt, mit Wasser alkalifrei gewaschen und getrocknet. Man erhält 128 Teile (91% der Theorie) 8-[4-Methylphenyl]-N,N'-dicarbmethoxy-2,3,7-triaza-6-oxa-tricyclo-[5.2.1.$0^{5,9}$]-deca-7-en vom Fp 163°C.

b)

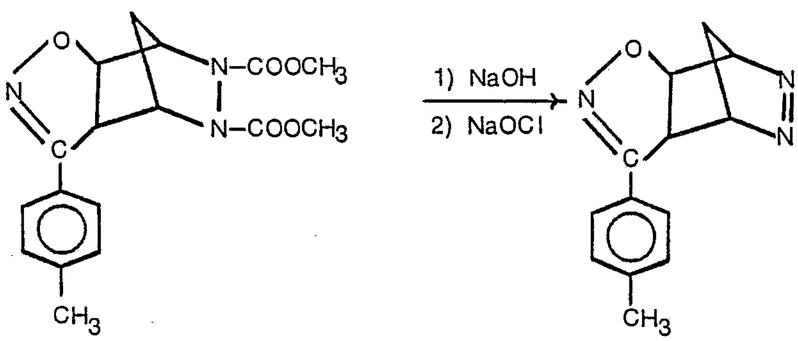


40 Teile 8- 4-Methylphenyl-N,N'-dicarbmethoxy-2,3,7-triaza-6-oxa-tricyclo-[5.2.1.$0^{5,9}$]-deca-7-en werden bei 68°C 4 Stunden mit 45 Teilen 50-gewichtsprozentiger, wäßriger Natronlauge und 400 Teilen Methanol gerührt. Danach gibt man bei 68°C 100 Teile 13-gewichtsprozentige, wäßrige NaOCl-Lösung innerhalb von einer Stunde unter Rühren zu und rührt noch eine Stunde bei 68°C nach. Anschließend versetzt man das auf 25°C abgekühlte Reaktionsgemisch mit 500 Teilen Wasser, saugt den Niederschlag ab, wäscht ihn mit Wasser alkalifrei und trocknet ihn. Man erhält 23 Teile (88,5% der Theorie) 8-[4-Methylphenyl]-2,3,7-triaza-6-oxa-tricyclo-[5.2.1.$0^{5,9}$]-deca-2,7-dien vom Fp 141°C.

Beispiele 4 bis 15

Entsprechend den Beispielen 1 oder 2 werden die in folgender Tabelle aufgeführten Endstoffe I hergestellt.

TABELLE

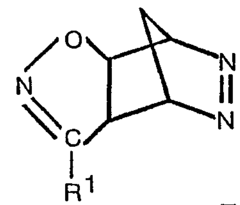

| Beispiel | $R^1$ | Umsetzung analog Beispiel | Teile Ausgangsstoff (II) | Teile Ausgangsstoff (III) |
|---|---|---|---|---|
| 4 | $CH_3-CH_2-$ | 1 | 71 | 212 |
| 5 | $CH_3-(CH_2)_2-$ | 1 | 85 | 212 |
| 6 | $CH_3-CH(CH_3)-$ | 1 | 85 | 212 |
| 7 | $(CH_3)_3C-$ | 1 | 100 | 212 |
| 8 | $HOCH_2-C(CH_3)_2-$ | 1 | 115 | 212 |
| 9 | $CH_3-(CH_2)_3-CH(C_2H_5)-$ | 1 | 141 | 212 |
| 10 | 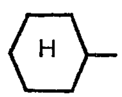 | 1 | 125 | 212 |
| 11 | $C_6H_5-CH_2-$ | 1a; 3b | 133 | 212 |
| 12 | $p-Cl-C_6H_4-$ | 2 | 61 | 85 |
| 13 | $p-CH_3O-C_6H_4-$ | 1 | 149 | 212 |
| 14 | 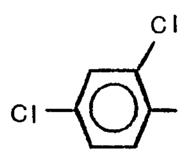 | 1 | 188 | 212 |
| 15 | 1-Naphthyl- | 1a; 3b | 169 | 212 |

TABELLE (Fortsetzung)

| Beispiel | Stoff (IV)<br>Ausbeute in % der Theorie | Fp °C; spektrale Werte | Endstoff (I)<br>Ausbeute in % der Theorie | Fp °C |
|---|---|---|---|---|
| 4 | 89 | 105 | 90 | 5 |
| 5 | 95 | 102 | 88 | 10 |
| 6 | 91 | 112 | 82 | 30 |
| 7 | 92 | $^1$H–NMR($\delta$ in ppm) 1.2(s,9H); 1.7(s, 2H); 3,6(d,1H); 4.7(m,3H) | 93 | 73 |
| 8 | 78 | $^1$H–NMR($\delta$ in ppm) 1.2(s,6H); 1.8(s,2H); 3.6(m,4H);3.8(s,6H); 4.8(m,3H) | 74 | 75 |
| 9 | 87 | $^1$H–NMR($\delta$ in ppm) 0.82–2.0(m,16H); 2.5(m,1H); 3.6(d,1H); 3.8(s,6H); 4.7(m,3H) | 79 | 8 |
| 10 | 93 | 130 | 88 | 83 |
| 11 | 74 | 150 | 79 | 68 |
| 12 | 96 | 176 | 87 | 138 |
| 13 | 85 | 192 | 89 | 154 |
| 14 | 92 | 148 | 93 | 112 |
| 15 | 88 | 180 | 85 | 118 |

**Patentanspruch**

Verfahren zur Herstellung von Isoxazolino-azo-verbindungen der Formel (I)

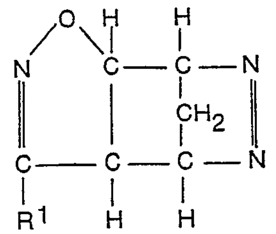

I

worin $R^1$ ein Wasserstoffatom, einen gegebenenfalls durch eine Hydroxygruppe substituierten Alkylrest mit 1 bis 18 Kohlenstoffatomen, einen Cycloalkylrest mit 3 bis 8 Kohlenstoffatomen, einen Aralkylrest oder Alkylarylrest mit jeweils 7 bis 12 Kohlenstoffatomen oder einen unsubstituierten oder einen Phenyl- oder Naphthylrest bezeichnet, der 1—3 fach durch Fluor, Brom, Chlor, Nitro, Trifluormethyl, Hydroxy, Dialkylamino, Alkoxy und/oder durch Fluor substituiertes Alkoxy mit jeweils 1 bis 4 Kohlenstoffatomen je Alkylgruppe substituiert sein kann, dadurch gekennzeichnet, daß in man einem ersten Schritt Nitriloxide der Formel (II)

$$R^1CNO \qquad II$$

worin $R^1$ die vorgenannte Bedeutung besitzt, mit N,N'-Dicarbalkoxy-2,3-diaza-bicyclo-[2,2,1]-hept-5-enen der Formel (III)

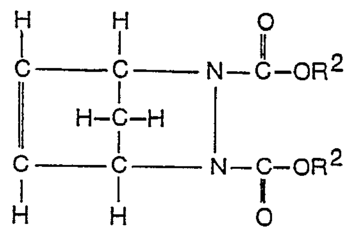

III

worin die einzelnen Reste $R^2$ gleich oder verschieden sein können und jeweils ein Wasserstoffatom oder einen gegebenenfalls durch eine Hydroxygruppe substituierten Alkylrest mit 1 bis 18 Kohlenstoffatomen bedeuten, umsetzt und in dann einem 2. Schritt die so erhaltenen N,N'-Dicarbalkoxy-isoxazolino-hydrazoverbindungen der Formel (IV)

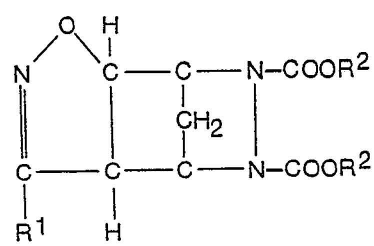

IV

worin $R^1$ und $R^2$ die vorgenannte Bedeutung besitzen, in Gegenwart eines Lösungsmittels, bestehend aus Wasser, Alkanolen und/oder Cycloalkanolen, und bei 40 — 100°C mit 4—25 Äquivalenten einer basischen Alkaliverbindung behandelt und gleichzeitig oder anschließend mit Alkalihypohalogeniten oder Wasserstoffperoxid oxidiert.

**Revendication**

Procédé pour la préparation de composés isoxazolino-axoïques de formule

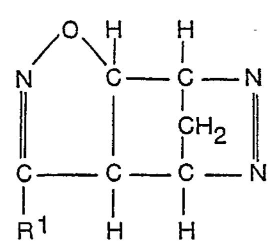

I

dans laquelle $R^1$ désigne un atome d'hydrogène, un radical alcoyle à 1—18 atomes de carbone, éventuellement substitué par un groupe hydroxy, un radical cycloalcoyle à 3— 8 atomes de carbone, un radical aralcoyle ou alcoylaryle à 7—12 atomes de carbone ou un radical phényle ou naphtyle qui peut être non substitué ou substitué 1 à 3 fois par un fluor, un brome, un chlore, un nitro, un trifluorométhyle, un hydroxy, un dialcoylamino, un alcoxy et/ou un alcoxy substitué par un fluor, comportant 1 à 4 atomes de carbone par groupe alcoyle, caractérisé en ce que, dans un premier temps, on fait réagir des oxydes de nitrile de formule (II)

$$R^1CNO \qquad (II)$$

dans laquelle $R^1$ a la signification donnée ci-dessus, avec des N,N'-dicarbalcoxy-2,3-diaza-bicyclo-[2,2,1]-heptènes-5 de formule (III)

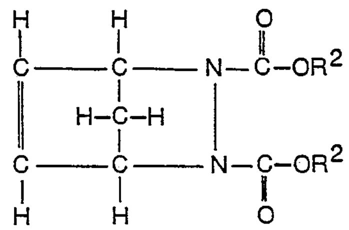

III

dans laquelle les différents radicaux $R^2$ peuvent être semblables ou dissemblables et représentent chacun un atome d'hydrogène ou un radical alcoyle à 1—18 atomes de carbone, éventuellement substitué par un groupe hydroxy, puis, dans un second temps, on traite les composés N,N'-dicarbalcoxy-isoxazolino-hydrazoïques de formule (IV) ainsi obtenus

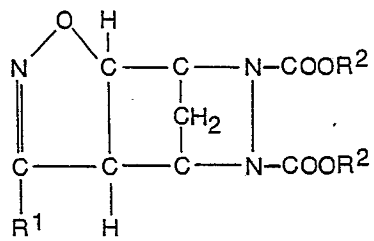

IV

dans laquelle $R^1$ et $R^2$ ont les significations données ci-dessus, en présence d'un solvant constitué par l'eau, des alcanols et/ou des cyclo-alcanols et à 40 — 100°C, par 4 à 25 équivalents d'un composé alcalin basique et, simultanément ou consécutivement on les oxyde avec des hypohalogénites alcalins ou le peroxyde d'hydrogène.

**Claim**

A process for the preparation of isoxazolino-azo compounds of the formul (I)

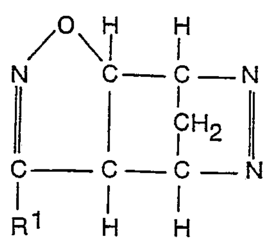

I

where $R^1$ is hydrogen, an optionally hydroxyl-substituted alkyl of 1 to 18 carbon atoms, cycloalkyl of 3 to 8 carbon atoms, aralkyl or alkylaryl each having 7 to 12 carbon atoms, phenyl or naphthyl which is unsubstituted or mono-, di- or trisubstituted by fluorine, bromine, chlorine, nitro, trifluoromethyl, hydroxyl, dialkylamino, alkoxy and/or fluorinesubstituted alkoxy, where alkyl is in each case of 1 to 4 carbon atoms, wherein in a first step nitrile oxides of the formula (II)

$$R^1—CNO \qquad II,$$

where $R^1$ has the above meanings, are reacted with N,N'-dicarbalkoxy-2,3-diaza-bicyclo-[2,2,1]-hept-5-enes of the formula (III)

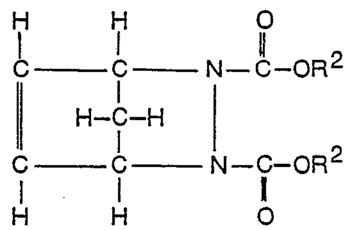

III

where the individual radicals $R^2$ may be identical or different and each denotes hydrogen or an optionally hydroxylsubstituted alkyl of 1 to 18 carbon atoms, and then in a second step the resulting N,N'-dicarbalkoxy-isoxazolinohydrazo compounds of the formula (IV)

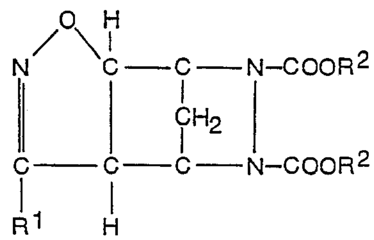

IV

where $R^1$ and $R^2$ have the above meanings, are treated in the presence of a solvent consisting of water, alkanols and/or cycloalkanols, and at 40 to 100°C with 4 to 25 equivalents of a basic alkali metal compound, and simultaneously or subsequently oxidized with alkali metal hypohalogenites or hydrogen peroxide.